Europäisches Patentamt

European Patent Office

Office européen des brevets

(19)

(11) **EP 0 564 843 B1**

(12) **EUROPÄISCHE PATENTSCHRIFT**

(45) Veröffentlichungstag und Bekanntmachung des Hinweises auf die Patenterteilung:
09.10.1996 **Patentblatt 1996/41**

(51) Int. Cl.⁶: **A61B 6/00**

(21) Anmeldenummer: 93104059.6

(22) Anmeldetag: **12.03.1993**

(54) **Verfahren zur Steuerung der Kompressionskraft bei einem Röntgengerät für Mammografieuntersuchungen und eine Anordnung zur Durchführung des Verfahrens**

Method to control the compression force of an X-Ray examining device for use in mammography and a device for use when carrying out this method

Procédé de contrôle de la force de compression aux mammographes et dispositif pour la mise en oeuvre de ce procédé

(84) Benannte Vertragsstaaten:
**DE FR GB IT SE**

(30) Priorität: **10.04.1992 SE 9201157**

(43) Veröffentlichungstag der Anmeldung:
**13.10.1993 Patentblatt 1993/41**

(73) Patentinhaber: **Siemens Elema AB**
**171 95 Solna 1 (SE)**

(72) Erfinder: **Thunberg, Stefan**
**S-118 58 Stockholm (SE)**

(56) Entgegenhaltungen:
EP-A- 0 144 670          DE-B- 1 942 490
US-A- 3 971 950          US-A- 3 991 316
US-A- 4 658 409

## Beschreibung

Die Erfindung betrifft ein Verfahren zur Steuerung der Kompressionskraft bei einem Röntgengerät für Mammografieuntersuchungen, bei dem eine Brust, die abgebildet werden soll, mit erhöhter Kompressionskraft zwischen einer Druckplatte und einem Objekttisch zusammengepresst wird.

Damit eine gute Bildqualität bei Röntgenaufnahmen von Brüsten erhalten wird, ist es notwendig, die Brust optimal zu komprimieren, um die Menge der Sekundärstrahlung vom Brustgewebe auf ein Minimum zu beschränken. Eine solche Sekundärstrahlung trägt zu einer Verschlechterung der Bildqualität bei, die aber mit abnehmender Brustdicke verringert wird.

Ein in der Einleitung erwähntes Verfahren, die Brust eines Patienten zu komprimieren, ist durch die EP-A 0 435 837 bekannt. Hier wird die Kompressionskraft erhöht,indem die Druckplatte mit einer gleichmässigen Geschwindigkeit bis zu einer vorbestimmten Lage gefahren wird. Danach wird die Druckplatte mit einer abnehmenden Geschwindigkeit bis zu einem vom Operatör im voraus eingestellten nicht variablen Endwert der Kompressionskraft gefahren. Der Nachteil des Verfahrens ist, dass der im voraus eingestellte Wert nicht an alle Brustgrössen angepasst ist, was zu einer zu grossen oder zu kleinen durch die Druckplatte auf die Brust ausgeübten Kompressionskraft führen kann. Ist die Brust zu klein, kann der Patient aufgrund einer zu grossen Kompressionskraft auf die Brust Schmerz erleben. Ist die Brust gross, ist die Kompressionskraft nicht immer ausreichend gross, um eine gute Bildqualität zu erhalten.

Der Erfindung liegt die Aufgabe zugrunde, ein Verfahren der eingangs genannten Art und eine Anordnung zur Durchführung des Verfahrens zu schaffen, bei denen ein optimaler Kompressionsdruck in jedem einzelnen Fall der Brustdicke angepasst ist, um auf diese Weise immer eine hohe Bildqualität zu erhalten und in gewissen Fällen einen unnötigen Schmerz beim Patienten zu vermeiden.

Diese Aufgabe ist erfindungsgemäss dadurch gelöst, dass die Kompressionskraft der Druckplatte, mit der diese gegen die Brust gedrückt wird, durch die Brustdicke derart gesteuert wird, dass die Erhöhung der Kompressionskraft abgebrochen wird, wenn die Änderung der Brustdicke einen einstellbaren Wert unterschreitet. Die Kompressionskraft wird hierdurch mit Rücksicht auf die Variationen der Brustdicke gesteuert, so dass die Erhöhung des Kompressionsdruckes abgebrochen wird, wenn die Dicke der komprimierten Brust nicht nennenswert verändert wird. Auf diese Weise wird die Brust nie einem unnötig hohen oder einem zu niedrigen Kompressionsdruck ausgesetzt, wobei immer ein hochqualitatives Röntgenbild erhalten wird.

In einer vorteilhaften Ausbildung der Erfindung wird vorgeschlagen, dass die Erhöhung der Kompressionskraft abgebrochen wird, wenn die Änderung der Brustdicke im Verhältnis zur Änderung der Kompressionskraft einen einstellbaren zweiter Wert unterschreitet. Der einstellbare Wert nach dem Verfahren ist vorteilhafterweise Null.

In einer Weiterbildung der Erfindung wird auch eine Anordnung zur Durchführung des Verfahrens vorgeschlagen,die ein erstes Mittel zur Bestimmung des Abstandes zwischen der Druckplatte und dem Objekttisch und damit auch zur Bestimmung der Brustdicke bei erhöhter Kompressionskraft sowie eine Steuervorrichtung umfasst, die die Erhöhung der Kompressionskraft abbricht, wenn die Änderung des erwähnten Abstandes einen einstellbaren Wert unterschreitet. Hierdurch wird durch einfache Mittel, unabhängig von der Brustdicke des Patienten, immer eine optimale Kompressionskraft auf die Brust erreicht, was die bereits erwähnten Vorteile gibt.

In einer vorteilhaften Ausgestaltung der Anordnung, bei der die Druckplatte flexibel ist und in einer definierten Weise mit erhöhter Kompressionskraft gebogen wird, wird vorgeschlagen, dass der Abstand, der mit Hilfe des ersten Mittels bestimmt wird, mit einem Wert korrigiert wird, so dass die Biegung der Druckplatte kompensiert wird. Die Druckplatte wird mit einer erhöhten Kompressionskraft derart gebogen, dass der Abstand zwischen deren freiem Ende und dem Objekttisch grösser wird als der Abstand zwischen dem eingespannten Ende der Druckplatte und dem Objekttisch. Der erwähnte Wert soll der Unterschied zwischen den erwähnten Abständen kompensieren.

Im Hinblick auf eine weitere Ausbildung der Erfindung wird vorgeschlagen, dass die Anordnung ein zweites Mittel zur Messung der Kompressionskraft aufweist, dessen Messwert zusammen mit dem Abstandswert, der mit Hilfe des ersten Mittels bestimmt wird, einem dritten Mittel zugeführt werden, das den Gradienten des Abstandes über der Kompressionskraft ($\frac{\Delta a}{\Delta b}$) bestimmt, und dass der Gradientenwert mit dem eingestellten zweiten Wert verglichen wird, wobei die Steuervorrichtung die Erhöhung der Kompressionskraft unterbricht, wenn der Gradientenwert den eingestellten Wert unterschreitet.

Eine vorteilhafte Weiterbildung der Erfindung ergibt sich daraus, dass das zweite Mittel zur Messung der Kompressionskraft und das erste Mittel zur Bestimmung des Abstandes in einer Hubvorrichtung für die Druckplatte angebracht sind. Es ist von Vorteil, wenn diese Mittel im Anschluss zur Druckplatte angebracht sind.

Im Hinblick auf eine vorteilhafte Weiterbildung der Erfindung wird vorgeschlagen, dass die Werte für den Abstand und für die Kompressionskraft abgetastet werden und einem Mikroprozessor, der in der Steuervorrichtung angebracht ist, zugeführt werden, wobei der Mikroprozessor den Gradientenwert, möglicherweise mit Rücksicht auf die Biegung der Druckplatte, berechnet und den Gradientenwert mit einem Referenzwert vergleicht, der dem eingestellten zweiten Wert entspricht, sowie ein Steuersignal erzeugt, das die Erhöhung der Kompressionskraft abbricht, wenn der Gradientenwert den eingestellten Wert unterschreitet.

Die Erfindung ist nachfolgend anhand eines in den Zeichnungen dargestellten Ausführungsbeispiels näher erläutert. Es zeigen:

FIG 1        eine Seitenansicht eines Röntgengerätes für Mammografieuntersuchungen nach der Erfindung und

FIG 2 und 3  Diagramme, die die Bewegung der Druckplatte im Vergleich zum Objekttisch bzw. zur Brustdicke als eine Funktion der Kompressionskraft darstellen.

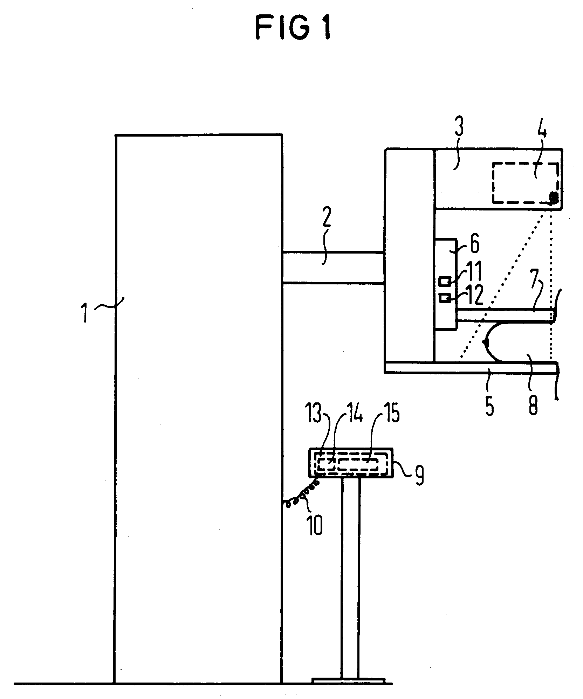

In der FIG 1 ist ein Röntgengerät für Mammografieuntersuchungen mit einem Stativ 1 gezeigt, das über eine Welle 2 ein Gehäuse 3 trägt, das eine Röntgenröhre 4 beinhaltet. An dem Gehäuse 3 ist ein Objekttisch 5, der gleichzeitig als eine Kassettenhalterung für Röntgenfilme dient, befestigt. An dem Gehäuse 3 ist ferner eine Hubvorrichtung 6 für eine vertikal verschiebbare Druckplatte 7 zur Komprimierung einer Brust 8 angebracht. Die Bewegung der Druckplatte 7 kann z.B. mittels eines Pults 9, das über ein Kabel 10 an dem Röntgengerät angeschlossen ist, gesteuert werden. Die Steuerung der Druckplatte 7 erfolgt mit Hilfe von Messmitteln 11, 12, die in der Hubvorrichtung 6 angebracht sind. Die Messmittel 11, 12 sind teils für die Messung der Kompressionskraft und teils für die Bestimmung bzw. Messung des Abstandes zwischen der Druckplate 7 und dem Objekttisch 5, d.h. für die Messung bzw. Bestimmung der Brustdicke vorgesehen. Diese Messmittel, die ein Abstandsmesser 11 und ein Kompressionskraftmesser 12 sind, sind an einer in dem Pult 9 angeordneten Steuervorrichtung 13 angeschlossen. Bei einer Röntgenaufnahme einer Brust 8, die auf dem Objekttisch 5 appliziert ist, drückt die Platte 7 die Brust gegen den Objekttisch 5. Die Erhöhung der Kompressionskraft wird unterbrochen, wenn die Änderung der Brustdicke im Vergleich zur Änderung der Kompressionskraft einen im voraus eingestellten Wert, z.B. 0, unterschreitet.

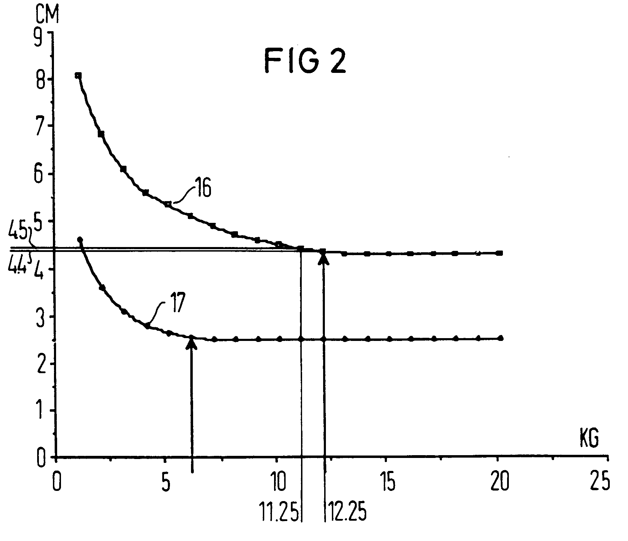

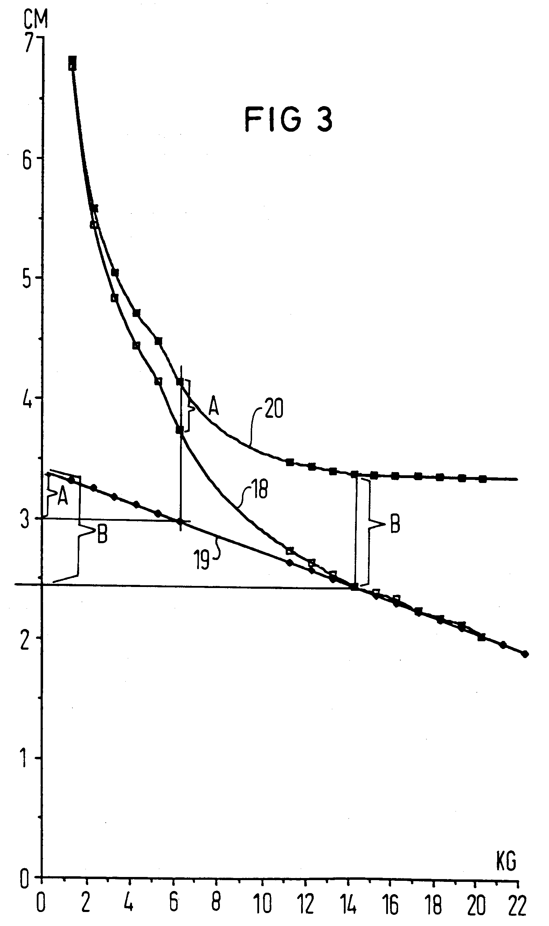

Die vom Abstandsmesser 11 und vom Kompressionsmesser 12 erhaltenen Messergebnissen werden kontinuierlich oder mit kurzen Zeitintervallen einem in der Steuervorrichtung 13 angeordneten Mittel 14 zugeführt, das den Gradiente des Abstandes zwischen der Druckplatte 7 und dem Objekttisch 5 über der Kompressionskraft ($\frac{\Delta a}{\Delta b}$) bestimmt. Dies wird in Verbindung mit den FIG 2 und 3 näher beschrieben. Die Steuervorrichtung 13 umfasst auch einen Komparator 15, der den Gradientenwert mit einem Referenzwert vergleicht, der dem erwähnten im voraus eingestellten Wert, der vorzugsweise 0 sein kann, entspricht sowie ein Steuersignal erzeugt, das der Hubvorrichtung 6 der Druckplatte 7 zugeführt wird und das die Erhöhung der Kompressionskraft unterbricht, wenn der Gradientenwert den Referenzwert unterschreitet. Das Mittel 14 und der Komparator 15 können mit Hilfe eines Mikroprozessors realisiert werden.

In der FIG 2 ist ein Diagramm mit einem Koordinatensystem gezeigt, bei dem die Abzisse die Kompressionskraft in Kg und die Ordinate die Brustdicke in cm angibt. Die obere Kurve 16 zeigt die Komprimierung einer Brust, deren Dicke bei einer Kompressionskraft von etwa 2 Kg etwa 8cm ist. Die Messergebnisse vom Abstandsmesser 11 und vom Kompressionskraftmesser 12 werden, wie bereits beschrieben, der Steuervorrichtung 13 zugeführt. Wenn der Gradientenwert der Kurve 16 den im voraus eingestellten Wert unterschreitet, wird die Erhöhung der Kompressionskraft abgebrochen. In diesem Ausführungsbeispiel erfolgt die Unterbrechung, wenn sich der Abstand zwischen der Druckplatte 7 und dem Objekttisch 5 und damit die Brustdicke bei einer Kompressionserhöhung von etwa 1 Kg nur etwa 1mm verringert hat. Die Kompressionskraft auf die Brust 8 bleibt bei etwa 12,25 Kg stehen. Die Kurve 16 zeigt, dass bei einer weiteren Erhöhung der Kompressionskraft die Verringerung der Brustdicke unerheblich ist. Eine solche Erhöhung der Kompressionskraft würde dem Patienten nur Schmerz bereiten und keine Verbesserung der Bildqualität ergeben. Die Kurve 17 zeigt die Komprimierung einer verhältnismässig kleinen Brust, deren Dicke bei einer Kompressionskraft von etwa 2 Kg etwa 4,5 cm ist. In diesem Fall wird die Erhöhung der Kompressionskraft, wie beschrieben, bei 6 Kg unterbrochen. Auch diese Kurve zeigt, dass eine Erhöhung des Kompressionsdruckes die Brustdicke nicht nennenswert verringert. In der Fig 2 ist der Unterschied der Endwerte der optimalen Kompressionskraft bei verschiedenen Brustdicken deutlich dargestellt. Es ist demnach nicht sinnvoll, die Kompressionskraft unabhängig von der Brustdicke auf einen im voraus bestimmten Endwert einzustellen.

In der FIG 3 ist ein weiteres Diagramm mit einem Koordinatensystem gezeigt, bei dem die Abzisse die Kompressionskraft in Kg und die Ordinate den Abstand zwischen der Druckplatte 7 und dem Objekttisch 5 in cm angibt. Die Kurve 18 in diesem Diagramm zeigt die Form, die eine Kurve erhält, wenn die Druckplatte 7 flexibel ist und in einer definierten Weise mit erhöhter Kompressionskraft gegen die Brust 8 gebogen wird. Die gerade Linie 19 zeigt die Eigenbiegung der Druckplatte 7. Um die Biegung der Druckplatte 7 zu kompensieren, wird der Abstand, der mit Hilfe des Abstandsmessers 11 bestimmt wird, mittels eines Kompensationswertes korrigiert. Wenn die Druckplatte 7 z.B. eine Biegung A aufweist, wird der Wert des Abstandsmessers 11 mit demselben Wert A kompensiert, und wenn die Druckplatte 7 z.B. eine Biegung B aufweist, wird der Wert des Abstandsmesser 11 mit demselben Wert B kompensiert. Somit wird eine Kurve 20 erhalten, und wenn der Gradientenwert der Kurve 20 den im voraus eingestellten Wert unterschreitet, wird die Erhöhung der Kompressionskraft wie bereits beschrieben abgebrochen.

Im Rahmen der Erfindung kann, um die Mittel zur Kompensierung der Biegung der Druckplatte 7 zu vermeiden, der Abstandsmesser 11 in der Mitte der Druckplatte 7 oder an deren freien Ende angebracht sein.

## Bezugszeichenliste

| | |
|---|---|
| 1 | Stativ |
| 2 | Welle |
| 3 | Gehäuse |
| 4 | Röntgenröhre |
| 5 | Objekttisch |
| 6 | Hubvorrichtung |
| 7 | Druckplatte |
| 8 | Brust |
| 9 | Pult |
| 10 | Kabel |
| 11 | Erstes Mittel, Messmittel, Abstandsmesser |
| 12 | Zweites Mittel, Messmittel, Kompressionskraftmesser |
| 13 | Steuervorrichtung |
| 14 | Drittes Mittel, Messmittel |
| 15 | Mikroprozessor, Komparator |
| 16,17,18,20 | Kurve |
| 19 | Linie |
| A, B | Biegung, Kompensationswert |

## Patentansprüche

1. Verfahren zur Steuerung der Kompressionskraft bei einem Röntgengerät für Mammografieuntersuchungen, bei dem eine Brust, die abgebildet werden soll, mit erhöhter Kompressionskraft zwischen einer Druckplatte und einem Objekttisch zusammengepresst wird, **dadurch gekennzeichnet**, dass die Kompressionskraft der Druckplatte (7), mit der diese gegen die Brust (8) gedrückt wird, durch die Brustdicke derart gesteuert wird, dass die Erhöhung der Kompressionskraft abgebrochen wird, wenn die Änderung der Brustdicke einen einstellbaren Wert unterschreitet.

2. Verfahren nach Anspruch 1, **dadurch gekennzeichnet**, dass die Erhöhung der Kompressionskraft abgebrochen wird, wenn die Änderung der Brustdicke im Verhältnis zur Änderung der Kompressionskraft einen einstellbaren Referenzwert unterschreitet.

3. Verfahren nach Anspruch 2, **dadurch gekennzeichnet**, dass der einstellbare Referenzwert Null ist.

4. Anordnung zur Durchführung des Verfahrens nach einem der Ansprüche 1 bis 3, **gekennzeichnet durch** ein erstes Mittel (11) zur Bestimmung des Abstandes zwischen der Druckplatte (7) und dem Objekttisch (5) und damit auch zur Bestimmung der Brustdicke bei erhöhter Kompressionskraft sowie eine Steuervorrichtung (13), die die Erhöhung der Kompressionskraft abbricht, wenn die Änderung des erwähnten Abstandes einen einstellbaren Wert unterschreitet.

5. Anordnung nach Anspruch 4, bei der die Druckplatte flexibel ist und in einer definierten Weise mit erhöhter Kompressionskraft gebogen wird, **dadurch gekenn** dass der Abstand, der mit Hilfe des ersten Mittels (11) bestimmt wird, mit einem Wert korrigiert wird, so dass die Biegung der Druckplatte (7) kompensiert wird.

6. Anordnung nach Anspruch 4 oder 5, **gekennzeichnet durch** ein zweites Mittel (12) zur Messung der Kompressionskraft, dessen Messwert zusammen mit dem Abstandswert, der mit Hilfe des ersten Mittels (11) bestimmt wird, einem dritten Mittel (14) zugeführt werden, das den Gradienten des Abstandes über der Kompressionskraft ($\frac{\Delta a}{\Delta b}$) bestimmt, und dass der Gradientenwert mit einem eingestellten Referenzwert verglichen wird, wobei die Steuervorrichtung (13) die Erhöhung der Kompressionskraft unterbricht, wenn der Gradientenwert den eingestellten Wert unterschreitet.

7. Anordnung nach einem der Ansprüche 4 bis 6, **dadurch gekennzeichnet**, dass das zweite Mittel (12) zur Messung der Kompressionskraft und das erste Mittel (11) zur Bestimmung des Abstandes in einer Hubvorrichtung (6) für die Druckplatte (7) angebracht sind.

8. Anordnung nach einem der Ansprüche 4 bis 7, **dadurch gekennzeichnet**, dass die Werte für den Abstand und für die Kompressionskraft abgetastet werden und einem Mikroprozessor (15), der in der Steuervorrichtung (13) angebracht ist, zugeführt werden, wobei der Mikroprozessor (15) den Gradientenwert, möglicherweise mit Rücksicht auf die Biegung der Druckplatte (7), berechnet und den Gradientenwert mit einem Referenzwert vergleicht, der dem eingestellten zweiten Wert entspricht, sowie ein Steuersignal erzeugt, das die Erhöhung der Kompressionskraft abbricht, wenn der Gradientenwert den eingestellten Wert unterschreitet.

## Claims

1. Method for controlling the compression force in an X-ray apparatus for mammography examinations, in which a breast which is intended to be depicted is compressed between a pressure plate and an object table with elevated compression force, characterized in that the compression force of the pressure plate (7) with which the latter is pressed against the breast (8) is controlled by the breast thickness in such a manner that the increasing of the compression force is interrupted when the alteration in the breast thickness falls below an adjustable value.

2. Method according to Claim 1, characterized in that the increasing of the compression force is interrupted when the alteration in the breast thickness, as a ratio to the alteration in the compression force, falls below an adjustable reference value.

3. Method according to Claim 2, characterized in that the adjustable reference value is zero.

4. Arrangement for carrying out the method according to one of Claims 1 to 3, characterized by a first means (11) for determining the distance between the pressure plate (7) and the object table (5) and hence also for determining the breast thickness at elevated compression force, as well as a control device (13) which interrupts the increasing of the compression force when the alteration of the said distance falls below an adjustable value.

5. Arrangement according to Claim 4, in which the pressure plate is flexible and is bent in a defined manner at elevated compression force, characterized in that the distance, which is determined with the aid of the first means (11), is corrected using a value in such a way that the bending of the pressure plate (7) is compensated.

6. Arrangement according to Claim 4 or 5, characterized by a second means (12) for measuring the compression force, the measured value of which together with the distance value, which is determined with the aid of the first means (11), are fed to a third means (14) which determines the gradient of the distance over the compression force ($\Delta a / \Delta b$), and in that the gradient value is compared with a set reference value, the control device (13) interrupting the increasing of the compression force when the gradient value falls below the set value.

7. Arrangement according to one of Claims 4 to 6, characterized in that the second means (12) for measuring the compression force and the first means (11) for determining the distance are fitted in a lifting device (6) for the pressure plate (7).

8. Arrangement according to one of Claims 4 to 7, characterized in that the values for the distance and for the compression force are sampled and are fed to a microprocessor (15), which is fitted in the control device (13), the microprocessor (15) calculating the gradient value, possibly taking into account the bending of the pressure plate (7), and comparing the gradient value with a reference value which corresponds to the set second value, and also generating a control signal which interrupts the increasing of the compression force when the gradient value falls below the set value.

## Revendications

1. Procédé de commande de la force de compression dans un appareil à rayons X pour des examens mammographiques, dans lequel on comprime une poitrine, qui doit être reproduite, par une force de compression augmentée, entre une plaque de pression et une table de support, caractérisé en ce que l'on commande, par l'épaisseur de la poitrine, la force de compression de la plaque (7) de pression, par laquelle on la presse contre la poitrine (8), de sorte à interrompre l'augmentation de la force de compression, lorsque la variation de l'épaisseur de la poitrine devient inférieure à une valeur réglable.

2. Procédé suivant la revendication 1, caractérisé en ce que l'on interrompt l'augmentation de la force de compression, lorsque la variation de l'épaisseur de la poitrine par rapport à la variation de la force de compression devient inférieure à une valeur de référence réglable.

3. Procédé suivant la revendication 2, caractérisé en ce que la valeur de référence réglable est zéro.

4. Dispositif de mise en oeuvre du procédé suivant l'une des revendications 1 à 3, caractérisé par un premier moyen (11) de détermination de la distance entre la plaque (7) de pression et la table (5) de support et ainsi également, de détermination de l'épaisseur de la poitrine lorsque la force de compression augmente, ainsi qu'un dispositif (13) de commande, qui interrompt l'augmentation de la force de compression, lorsque la variation de la distance mentionnée devient inférieure à une valeur réglable.

5. Dispositif suivant la revendication 4, dans lequel la plaque de pression est flexible et est fléchie d'une manière définie lorsque la force de compression est augmentée, caractérisé en ce que la distance, qui est déterminée à l'aide du premier moyen (11), est corrigée par une valeur, de sorte à compenser la flexion de la plaque (7) de pression.

6. Dispositif suivant la revendication 4 ou 5, caractérisé par un second moyen (12) de mesure de la force de compression, dont la valeur de mesure est envoyée, conjointement avec la valeur de la distance déterminée à l'aide du premier moyen (11), à un troisième moyen, qui détermine le gradient de la distance par apport à la force de compression ($\frac{\Delta a}{\Delta b}$) et la valeur du gradient est comparée à une valeur de référence réglée, le dispositif (13) de commande interrompant l'augmentation de la force de compression, lorsque la valeur du gradient devient inférieure à la valeur réglée.

7. Dispositif suivant l'une des revendications 4 à 6, caractérisé en ce que le second moyen (12) de mesure de la force de compression et le premier moyen (11) de détermination de la distance sont mis en place dans un dispositif (6) de levage de la plaque (7) de pression.

8. Dispositif suivant l'une des revendications 4 à 7, caractérisé en ce que les valeurs de la distance et de la force de compression sont échantillonnées et sont envoyées à un microprocesseur (15), qui est mis en place dans le dispositif (13) de commande, le microprocesseur (15) calculant la valeur du gradient, si possible en tenant compte de la flexion de la plaque (7) de pression et comparant la valeur du gradient à une valeur de référence, qui correspond à la seconde valeur réglée, et produisant un signal de commande, qui interrompt l'augmentation de la force de compression, lorsque la valeur du gradient devient inférieure à la valeur réglée.

# FIG 1

FIG 2

FIG 3